(19) Europäisches Patentamt
European Patent Office
Office européen des brevets

(11) **EP 4 321 034 A1**

(12) **EUROPEAN PATENT APPLICATION**
published in accordance with Art. 153(4) EPC

(43) Date of publication:
**14.02.2024 Bulletin 2024/07**

(21) Application number: **22784701.9**

(22) Date of filing: **07.04.2022**

(51) International Patent Classification (IPC):
***A23K 10/37*** *(2016.01)*   ***A23K 50/10*** *(2016.01)*

(52) Cooperative Patent Classification (CPC):
**A23K 10/37; A23K 50/10**

(86) International application number:
**PCT/JP2022/017226**

(87) International publication number:
**WO 2022/215719 (13.10.2022 Gazette 2022/41)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **08.04.2021   JP 2021065837**

(71) Applicants:
• **Tokuyama Corporation
Shunan-shi, Yamaguchi 745-8648 (JP)**
• **School Corporation, Azabu Veterinary
Medicine Educational Institution
Sagamihara-shi, Kanagawa 252-5201 (JP)**

(72) Inventors:
• **YAMADA, Kie
Shunan-shi, Yamaguchi 745-8648 (JP)**
• **INUI, Yoji
Shunan-shi, Yamaguchi 745-8648 (JP)**
• **KAWAI, Kazuhiro
Sagamihara-shi, Kanagawa 252-5201 (JP)**
• **KURUMISAWA, Tomomi
Sagamihara-shi, Kanagawa 252-5201 (JP)**

(74) Representative: **Kraus & Lederer PartGmbB
Thomas-Wimmer-Ring 15
80539 München (DE)**

(54) **METHANE GAS REDUCING AGENT**

(57)    Methane production from ruminant digestion is reduced by allowing a ruminant to be fed on feed containing unfermented coffee grounds.

EP 4 321 034 A1

**Description**

Technical Field:

The present invention relates to a novel methane reducing agent. More specifically, the present invention relates to a methane reducing agent for reducing methane production from ruminant digestion.

Background Art:

A large amount of food residues is generated from the food and food service industries and is disposed of by incineration or landfill under current circumstances. In particular, as coffee consumption increases globally, disposal of coffee grounds to be food residues has become an issue, which coffee grounds are difficult to dispose of as they are by incineration due to their high moisture and fiber contents.

As a measure to address this issue, an attempt has been made to turn coffee grounds into useful recyclable resources by bacterial fermentation.

For example, Patent Document 1 discloses a technique to turn coffee grounds into livestock feed with reduced caffeine by fermentation with Bacillus subtilis. With this technique, effective use for coffee grounds can be found as livestock feed with improved palatability.

Meanwhile, ruminants such as cows have four stomachs to repeat digestion ingested plants constantly over time. During the digestion process, hydrogen and carbon dioxide are produced as the plants and the like are degraded (fermented) by intragastric bacteria. Using the hydrogen and carbon dioxide, a group of archaea called methanogens produces methane. The thus-produced methane is released into the atmosphere as a burp.

Methane is estimated to be 25 times more potent than carbon dioxide in terms of its greenhouse effect, and burps from ruminants are said to account for 20% to 30% of methane in the atmosphere.

To prevent the greenhouse effect, it is required to reduce methane by burps from ruminants.

Patent Document 2 discloses a technique to reduce methane by using livestock feed obtained by fermenting coffee grounds by bacteriocin-producing lactic acid bacterium.

Prior Art Documents:

Patent Documents:

Patent Document 1: JP-A-2013-179917
Patent Document 2: WO 2018/003034

Summary of the Invention:

Problems to be solved by the Invention:

However, the livestock feed disclosed in Patent Document 1 requires time and effort for the fermentation treatment with bacteria. Further, no consideration is given to the reduction of methane in Patent Document 1.

The livestock feed disclosed in Patent Document 2 has a beneficial effect on the reduction of methane; however, it also requires time and effort for the fermentation treatment with bacteria as in Patent Document 1. Further, it is difficult to perform a stable treatment constantly using microorganisms including bacteria. Furthermore, this document fails to disclose how to directly measure methane; thus, it is not known how much reduction of methane is achieved.

Under the circumstances, there has been a demand for a method for reducing methane production from livestock (ruminant) digestion, reliably and easily without a complex treatment.

Means for solving the Problems:

In order to solve the aforementioned problem, the present inventors conducted an intensive study. They first found a method of directly measuring methane produced in the rumen of cows (ruminants) during the digestion of ingested feed and the like. Using this measurement method, they measured the amount of methane produced by cows that fed on unfermented coffee grounds as food residues. As a result, they found a surprising fact that the coffee grounds had a beneficial effect in reducing methane emissions from ruminants. Thus, the present invention has been completed.

The present invention provides a methane reducing agent containing unfermented coffee grounds.

The present invention also provides ruminant feed containing unfermented coffee grounds as a methane reducing agent.

In the ruminant feed of the present invention,

(1) the methane reducing agent is contained in an amount of 0.5 to 30 mass%; and
(2) the unfermented coffee grounds have a moisture content of 40 mass% or less.

The present invention further provides a method for reducing methane production from ruminant digestion, including allowing a ruminant to be fed on feed containing unfermented coffee grounds.

Effect of the Invention:

The present invention makes it possible to reduce methane production from ruminant (e.g., cow) digestion by feeding coffee grounds that are not fermented, namely, unfermented coffee grounds.

When ruminants eat plants and the like, the ingested food is first digested in the first stomach (the rumen) while being fermented by bacteria present in the rumen, which produces methane gas as described above. The methane reducing agent (unfermented coffee grounds) of the present invention is contained in feed. When this feed is ingested, the production of methane is suppressed presumably because the unfermented coffee grounds act on bacterial fermentation.

Mode for Carrying Out the Invention:

The coffee grounds for use in the present invention are not particularly limited. For reasons of availability, the coffee grounds may be obtained from, for example, instant coffee or coffee beverage manufactures, coffee shops, and the like.

The coffee grounds in the present invention may be subjected to any treatment other than a fermentation treatment. For example, the coffee grounds, which are high in moisture content, may be subjected to a known drying treatment.

According to said dry treatment, the coffee grounds are suitably dried to a moisture content of 40 mass% or less, more suitably 30 mass% or less, and still more suitably 15 mass% or less. Most suitably, the coffee grounds are dried to a moisture content of 5 to 15 mass%.

The drying temperature is not particularly limited, but is preferably, for example, 70°C or less, more preferably 50°C or less, and most preferably 40°C or less, from the viewpoint of preventing deterioration of the coffee grounds. The drying method is not particularly limited either.

The methane reducing agent of the present invention is unfermented coffee grounds. In terms of palatability, the methane gas reducing agent is preferably blended with livestock feed known per se. The resultant livestock feed blend may be used as feed for ruminants such as cows. The method of blending unfermented coffee grounds with livestock feed is not particularly limited, and a known blending method may be used.

Examples of known livestock feed include roughage such as grass, rice straw and silage; and concentrated feed such as grain (corn, rice, sorghum, barley, etc.), bran (wheat barn, rice bran, etc.), by-products (soybean oil cake, beet pulp, draff, soy pulp, etc.), and animal-based feed (fish meal, etc.)

The content of the methane reducing agent (unfermented coffee grounds) of the present invention in the above-described ruminant feed is not particularly limited, but is usually 0.5 to 30 mass%, in particular 1 to 20 mass%, more preferably 2 to 20 mass%, and still more preferably 2 to 15 mass%. If the ruminant feed contains the methane reducing agent in a smaller amount, the effect of reducing methane may be insufficient. If the ruminant feed contains the methane reducing agent in an excessive amount, ruminants may avoid eating the feed, which can lead to insufficient feeding.

The ruminant feed containing the methane reducing agent of the present invention can contain an additive for increasing the effect of reducing methane. Examples of the additive include a compound containing a metal such as an alkali metal, an alkali earth metal, or a transition metal. Among them, the additive is preferably a transition metal compound, more preferably an oxide or hydroxide of iron or copper, and particularly preferably an iron oxide. The additive is preferably added in an amount of 0.1 to 5 parts by mass per 100 parts by mass of the ruminant feed containing the methane reducing agent.

Examples:

In Experiments below, methane and carbon dioxide produced in the rumen of a cow during digestion were measured in the following manner.

A measuring tube was inserted into the rumen of a fistulated cow through the fistula. The aforementioned gases in the rumen were collected through the measuring tube and measured.

<Experiment 1>

14.3 parts by mass of unfermented coffee grounds (UCG) dried at 40°C (moisture content: 9%) were mixed with 100 parts by mass of livestock feed to prepare ruminant feed for reducing methane gas that contains 12.5 mass% of the unfermented coffee grounds. The thus-prepared ruminant feed was fed to a cow.

The cow was fed in the morning (7:50) and in the afternoon (15:00) in the following manner. Methane and carbon dioxide in the rumen were measured before the feeding and three hours after the feeding. The measured values are shown in Table 1.

  1st day: livestock feed as basic dist was fed both in the morning and in the afternoon.
  2nd to 4th day: The ruminant feed containing the unfermented coffee grounds was fed both in the morning and in the afternoon.
  5th day: Livestock feed as basic dist was fed both in the morning and in the afternoon.

The measured values for the ruminant feed (shown as "UCG") are the averages of the measured values obtained on the 2nd to 4th days.

The measured values for the livestock feed as basic dist (Control) that does not contain the unfermented coffee grounds are the averages of the measured values obtained on the 1st and 5th days.

In order to cancel the effect of carbon dioxide, the rate of methane was calculated using the following formula for the USG and the control, respectively. The results are shown in Table 1.

$$\text{Methane rate (\%)} = 100 \times \text{methane (vol\%)} / [(\text{methane (vol\%)} + \text{carbon dioxide (vol\%)}]$$

Further, the rate of methane reduction was calculated from the methane rate for the USG and the methane rate for the control using the following formula. The results are shown in Table 1.

$$\text{Reduction rate (\%)} = 100 \times (\text{control (\%)} - \text{USG (\%)}) / \text{control (\%)}$$

[Table 1]

| | Methane Rate (%) | | Reduction rate (%) |
|---|---|---|---|
| | UCG | Control | |
| Before morning feeding | 36.0% | 40.7% | 11.5% |
| Three hours after morning feeding | 35.5% | 41.1% | 13.6% |
| Before afternoon feeding | 31.9% | 39.2% | 18.5% |

As shown in Table 1, methane produced by the cow fed on the ruminant feed containing the unfermented coffee grounds (USG) (methane reducing agent) was reduced by about 12% to 19%.

<Experiment 2>

4.3 parts by mass of unfermented coffee grounds(USG) (moisture content: 10%) were used to prepare ruminant feed for reducing methane gas that contains 4.1 mass% of the unfermented coffee grounds (USG) . The thus-prepared ruminant feed was fed to a cow in the same manner as in Experiment 1. Methane and carbon dioxide were measured in the same manner as in Experiment

1. The results are shown in Table 2.

[Table 2]

| | Methane Rate (%) | | Reduction rate (%) |
|---|---|---|---|
| | UCG | Control | |
| Before morning feeding | 33.9% | 36.5% | 7.1% |
| Three hours after morning feeding | 26.9% | 31.0% | 13.1% |
| Before afternoon feeding | 29.0% | 29.0% | 0% |

As shown in Table 2, methane produced by the cow fed on the ruminant feed containing the unfermented coffee grounds (methane reducing agent) was reduced by about 7% to 13%.

**Claims**

1. A methane reducing agent containing unfermented coffee grounds.

2. Ruminant feed containing unfermented coffee grounds as a methane reducing agent.

3. The ruminant feed according to claim 2, wherein the methane reducing agent is contained in an amount of 0.5 to 30 mass%.

4. The ruminant feed according to claim 2, wherein the unfermented coffee grounds have a moisture content of 40 mass% or less.

5. A method for reducing methane production from ruminant digestion, containing allowing a ruminant to be fed on feed containing unfermented coffee grounds.

6. The method according to claim 5, wherein the feed contains the unfermented coffee grounds in an amount of 0.5 to 30 mass%.

7. The method according to claim 5, wherein the unfermented coffee grounds have a moisture content of 40 mass% or less.

## INTERNATIONAL SEARCH REPORT

| International application No. |
| --- |
| **PCT/JP2022/017226** |

### A. CLASSIFICATION OF SUBJECT MATTER

*A23K 10/37*(2016.01)i; *A23K 50/10*(2016.01)i
FI:   A23K10/37; A23K50/10

According to International Patent Classification (IPC) or to both national classification and IPC

### B. FIELDS SEARCHED

Minimum documentation searched (classification system followed by classification symbols)

A23K10/37; A23K50/10

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| A | WO 2018/003034 A1 (MENICON CO LTD) 04 January 2018 (2018-01-04)<br>entire text, all drawings | 1-7 |
| A | JP 2013-179917 A (MEIWA SANGYO) 12 September 2013 (2013-09-12)<br>entire text, all drawings | 1-7 |
| A | SANTOSO, B. et al. Effect of Secondary Metabolites in Beverage Industry Residues on Rumen Methane Emission. JIRCAS Working Report. 2013, no. 79, pp. 29-33<br>entire text, all drawings | 1-7 |
| A | 高橋敏能, 地域を中心とする新規飼料の開発と実用化の可能性を探る, 東北畜産学会報, 2013, vol. 32, no. 3, pp. 63-70<br>entire text, all drawings, (TAKAHASHI, Toshiyoshi. Tohoku Journal of Animal Science and Technology.), non-official translation (Development of new feed centered on the region and exploring the possibility of practical use) | 1-7 |

☐ Further documents are listed in the continuation of Box C.   ☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **24 May 2022** | **07 June 2022** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2022/017226**

| Patent document cited in search report | | | Publication date (day/month/year) | Patent family member(s) | | | Publication date (day/month/year) |
|---|---|---|---|---|---|---|---|
| WO | 2018/003034 | A1 | 04 January 2018 | EP 3479700 A1 entire text, all drawings CN 109640687 A | | | |
| JP | 2013-179917 | A | 12 September 2013 | (Family: none) | | | |

Form PCT/ISA/210 (patent family annex) (January 2015)

**EP 4 321 034 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2013179917 A
- WO 2018003034 A